# EUROPEAN PATENT SPECIFICATION

(11) **EP 3 108 257 B1**
(45) Date of publication and mention of the grant of the patent: **30.10.2019**
(21) Application number: 15705286.1
(22) Date of filing: 16.02.2015
(51) Int. Cl.: G01N 33/68

(54) **METHODS AND USES OF MITOFUSINS**
VERFAHREN UND VERWENDUNGEN VON MITOFUSINEN
PROCÉDÉS ET UTILISATIONS DE MITOFUSINE

(30) Priority: 17.02.2014 EP 14155312; 06.11.2014 EP 14192012
(43) Date of publication of application: 28.12.2016
(73) Proprietor: Société des Produits Nestlé S.A., 1800 Vevey (CH)
(72) Inventor: CANTO ALVAREZ, Carles, 1148 Cuarnens (CH); KULKARNI, Sameer, CH-1004 Lausanne (CH); ZORZANO, Antonio, E-08028 Barcelona (ES)
(74) Representative: Kamibayashi, Lynne
(86) International application number: PCT/EP2015/053169
(87) International publication number: WO 2015/121461

(56) References cited:
- WO-A1-98/55618
- WO-A1-2004/074482
- Y. EURA ET AL: "Identification of a novel protein that regulates mitochondrial fusion by modulating mitofusin (Mfn) protein function", JOURNAL OF CELL SCIENCE, vol. 119, no. 23, 1 December 2006 (2006-12-01), pages 4913-4925, XP055128175, ISSN: 0021-9533, DOI: 10.1242/jcs.03253
- MICHELA RANIERI ET AL: "Mitochondrial Fusion Proteins and Human Diseases", NEUROLOGY RESEARCH INTERNATIONAL, vol. 20, no. 2, 1 January 2013 (2013-01-01), pages 353-11, XP055128067, ISSN: 2090-1852, DOI: 10.1038/cddis.2011.117
- DIETRICH MARCELO O ET AL: "Mitochondrial Dynamics Controlled by Mitofusins Regulate Agrp Neuronal Activity and Diet-Induced Obesity", CELL, vol. 155, no. 1, 26 September 2013 (2013-09-26), pages 188-199, XP028729743, ISSN: 0092-8674, DOI: 10.1016/J.CELL.2013.09.004
- GAO C L ET AL: "Mitochondrial dysfunction is induced by high levels of glucose and free fatty acids in 3T3-L1 adipocytes", MOLECULAR AND CELLULAR ENDOCRINOLOGY, ELSEVIER IRELAND LTD, IE, vol. 320, no. 1-2, 14 May 2010 (2010-05-14), pages 25-33, XP026978604, ISSN: 0303-7207 [retrieved on 2010-02-06]

### SEQUENCE LISTING

<110> NESTEC S.A.
<120> Methods and Uses of Mitofusins
<130> G200785
<160> 12
<170> PatentIn version 3.5
<210> 1
   <211> 741
   <212> PRT
   <213> Homo sapiens
<400> 1
   Met Ala Glu Pro Val Ser Pro Leu Lys His Phe Val Leu Ala Lys Lys
   1               5                   10                  15
   Gly Ile Thr Ala Ile Phe Asp Gln Leu Leu Glu Phe Val Thr Glu Gly
               20                  25                  30
   Ser His Phe Val Glu Ala Thr Tyr Lys Asn Pro Glu Leu Asp Arg Ile
           35                  40                  45
   Ala Thr Glu Asp Asp Leu Val Glu Met Gln Gly Tyr Lys Asp Lys Leu
       50                  55                  60
   Ser Ile Ile Gly Glu Val Leu Ser Arg Arg His Met Lys Val Ala Phe
   65                  70                  75                  80
   Phe Gly Arg Thr Ser Ser Gly Lys Ser Ser Val Ile Asn Ala Met Leu
                   85                  90                  95
   Trp Asp Lys Val Leu Pro Ser Gly Ile Gly His Ile Thr Asn Cys Phe
               100                 105                 110
   Leu Ser Val Glu Gly Thr Asp Gly Asp Lys Ala Tyr Leu Met Thr Glu
           115                 120                 125
   Gly Ser Asp Glu Lys Lys Ser Val Lys Thr Val Asn Gln Leu Ala His
       130                 135                 140
   Ala Leu His Met Asp Lys Asp Leu Lys Ala Gly Cys Leu Val Arg Val
   145                 150                 155                 160
   Phe Trp Pro Lys Ala Lys Cys Ala Leu Leu Arg Asp Asp Leu Val Leu
                   165                 170                 175
   Val Asp Ser Pro Gly Thr Asp Val Thr Thr Glu Leu Asp Ser Trp Ile
               180                 185                 190
   Asp Lys Phe Cys Leu Asp Ala Asp Val Phe Val Leu Val Ala Asn Ser
           195                 200                 205
   Glu Ser Thr Leu Met Asn Thr Glu Lys His Phe Phe His Lys Val Asn
       210                 215                 220
   Glu Trp Leu Ser Lys Pro Asn Ile Phe Ile Leu Asn Asn Arg Trp Asp
   225                 230                 235                 240
   Ala Ser Ala Ser Glu Pro Glu Tyr Met Glu Asp Val Arg Arg Gln His
                   245                 250                 255
   Met Glu Arg Cys Leu His Phe Leu Val Glu Glu Leu Lys Val Ala Asn
               260                 265                 270
   Ala Leu Glu Ala Gln Asn Arg Ile Phe Phe Val Ser Ala Lys Glu Val
           275                 280                 285
   Leu Ser Ala Arg Lys Gln Lys Ala Gln Gly Met Pro Glu Ser Gly Val
       290                 295                 300
   Ala Leu Ala Glu Gly Phe His Ala Arg Leu Gln Glu Phe Gln Asn Phe
   305                 310                 315                 320
   Glu Gln Ile Phe Glu Glu Cys Ile Ser Gln Ser Ala Val Lys Thr Lys
                   325                 330                 335
   Phe Glu Gln His Thr Ile Arg Ala Lys Gln Ile Leu Ala Thr Val Lys
               340                 345                 350
   Asn Ile Met Asp Ser Val Asn Leu Ala Ala Glu Asp Lys Arg His Tyr
           355                 360                 365
   Ser Val Glu Glu Arg Glu Asp Gln Ile Asp Arg Leu Asp Phe Ile Arg
       370                 375                 380
   Asn Gln Met Asn Leu Leu Thr Leu Asp Val Lys Lys Lys Ile Lys Glu
   385                 390                 395                 400
   Val Thr Glu Glu Val Ala Asn Lys Val Ser Cys Ala Met Thr Asp Glu
                   405                 410                 415
   Ile Cys Arg Leu Ser Val Leu Val Asp Glu Phe Cys Ser Glu Phe His
               420                 425                 430
   Pro Asn Pro Asp Val Leu Lys Ile Tyr Lys Ser Glu Leu Asn Lys His
           435                 440                 445
   Ile Glu Asp Gly Met Gly Arg Asn Leu Ala Asp Arg Cys Thr Asp Glu
       450                 455                 460
   Val Asn Ala Leu Val Leu Gln Thr Gln Gln Glu Ile Ile Glu Asn Leu
   465                 470                 475                 480
   Lys Pro Leu Leu Pro Ala Gly Ile Gln Asp Lys Leu His Thr Leu Ile
                   485                 490                 495
   Pro Cys Lys Lys Phe Asp Leu Ser Tyr Asn Leu Asn Tyr His Lys Leu
               500                 505                 510
   Cys Ser Asp Phe Gln Glu Asp Ile Val Phe Arg Phe Ser Leu Gly Trp
           515                 520                 525
   Ser Ser Leu Val His Arg Phe Leu Gly Pro Arg Asn Ala Gln Arg Val
       530                 535                 540
   Leu Leu Gly Leu Ser Glu Pro Ile Phe Gln Leu Pro Arg Ser Leu Ala
   545                 550                 555                 560
   Ser Thr Pro Thr Ala Pro Thr Thr Pro Ala Thr Pro Asp Asn Ala Ser
                   565                 570                 575
   Gln Glu Glu Leu Met Ile Thr Leu Val Thr Gly Leu Ala Ser Val Thr
               580                 585                 590
   Ser Arg Thr Ser Met Gly Ile Ile Ile Val Gly Gly Val Ile Trp Lys
           595                 600                 605
   Thr Ile Gly Trp Lys Leu Leu Ser Val Ser Leu Thr Met Tyr Gly Ala
       610                 615                 620
   Leu Tyr Leu Tyr Glu Arg Leu Ser Trp Thr Thr His Ala Lys Glu Arg
   625                 630                 635                 640
   Ala Phe Lys Gln Gln Phe Val Asn Tyr Ala Thr Glu Lys Leu Arg Met
                   645                 650                 655
   Ile Val Ser Ser Thr Ser Ala Asn Cys Ser His Gln Val Lys Gln Gln
               660                 665                 670
   Ile Ala Thr Thr Phe Ala Arg Leu Cys Gln Gln Val Asp Ile Thr Gln
           675                 680                 685
   Lys Gln Leu Glu Glu Glu Ile Ala Arg Leu Pro Lys Glu Ile Asp Gln
   690 695 700
   Leu Glu Lys Ile Gln Asn Asn Ser Lys Leu Leu Arg Asn Lys Ala Val
   705 710 715 720
   Gln Leu Glu Asn Glu Leu Glu Asn Phe Thr Lys Gln Phe Leu Pro Ser
   725 730 735
   Ser Asn Glu Glu Ser
   740
<210> 2
   <211> 741
   <212> PRT
   <213> Mus musculus
<400> 2
   Met Ala Glu Thr Val Ser Pro Leu Lys His Phe Val Leu Ala Lys Lys
   1 5 10 15
   Ala Ile Thr Ala Ile Phe Gly Gln Leu Leu Glu Phe Val Thr Glu Gly
   20 25 30
   Ser His Phe Val Glu Ala Thr Tyr Arg Asn Pro Glu Leu Asp Arg Ile
   35 40 45
   Ala Ser Glu Asp Asp Leu Val Glu Ile Gln Gly Tyr Arg Asn Lys Leu
   50 55 60
   Ala Val Ile Gly Glu Val Leu Ser Arg Arg His Met Lys Val Ala Phe
   65 70 75 80
   Phe Gly Arg Thr Ser Ser Gly Lys Ser Ser Val Ile Asn Ala Met Leu
   85 90 95
   Trp Asp Lys Val Leu Pro Ser Gly Ile Gly His Thr Thr Asn Cys Phe
   100 105 110
   Leu Ser Val Glu Gly Thr Asp Gly Asp Lys Ala Tyr Leu Met Thr Glu
   115 120 125
   Gly Ser Asp Glu Lys Lys Ser Val Lys Thr Val Asn Gln Leu Ala His
   130 135 140
   Ala Leu His Met Asp Lys Asp Leu Lys Ala Gly Cys Leu Val His Val
   145 150 155 160
   Phe Trp Pro Lys Ala Lys Cys Ala Leu Leu Arg Asp Asp Leu Val Leu
                   165                 170                 175
   Val Asp Ser Pro Gly Thr Asp Val Thr Thr Glu Leu Asp Ile Trp Ile
               180                 185                 190
   Asp Lys Phe Cys Leu Asp Ala Asp Val Phe Val Leu Val Ala Asn Ser
           195                 200                 205
   Glu Ser Thr Leu Met Asn Thr Glu Lys His Phe Phe His Lys Val Asn
       210                 215                 220
   Glu Arg Leu Ser Lys Pro Asn Ile Phe Ile Leu Asn Asn Arg Trp Asp
   225                 230                 235                 240
   Ala Ser Ala Ser Glu Pro Glu Tyr Met Glu Asp Val Arg Arg Gln His
                   245                 250                 255
   Met Glu Arg Cys Leu His Phe Leu Val Glu Glu Leu Lys Val Val Ser
               260                 265                 270
   Pro Ser Glu Ala Arg Asn Arg Ile Phe Phe Val Ser Ala Lys Glu Val
           275                 280                 285
   Leu Asn Ser Arg Lys His Lys Ala Gln Gly Met Pro Glu Gly Gly Gly
       290                 295                 300
   Ala Leu Ala Glu Gly Phe Gln Ala Arg Leu Gln Glu Phe Gln Asn Phe
   305                 310                 315                 320
   Glu Gln Thr Phe Glu Glu Cys Ile Ser Gln Ser Ala Val Lys Thr Lys
                   325                 330                 335
   Phe Glu Gln His Thr Ile Arg Ala Lys Gln Ile Leu Asp Thr Val Lys
               340                 345                 350
   Asn Ile Leu Asp Ser Val Asn Val Ala Ala Ala Glu Lys Arg Val Tyr
           355                 360                 365
   Ser Met Glu Glu Arg Glu Asp Gln Ile Asp Arg Leu Asp Phe Ile Arg
       370                 375                 380
   Asn Gln Met Asn Leu Leu Thr Leu Asp Val Lys Lys Lys Ile Lys Glu
   385                 390                 395                 400
   Val Thr Glu Glu Val Ala Asn Lys Val Ser Cys Ala Met Thr Asp Glu
   405 410 415
   Ile Cys Arg Leu Ser Val Leu Val Asp Glu Phe Cys Ser Glu Phe His
   420 425 430
   Pro Thr Pro Ser Val Leu Lys Val Tyr Lys Ser Glu Leu Asn Lys His
   435 440 445
   Ile Glu Asp Gly Met Gly Arg Asn Leu Ala Asp Arg Cys Thr Asn Glu
   450 455 460
   Val Asn Ala Ser Ile Leu Gln Ser Gln Gln Glu Ile Ile Glu Asn Leu
   465 470 475 480
   Lys Pro Leu Leu Pro Ala Gly Ile Gln Asn Lys Leu His Thr Leu Ile
   485 490 495
   Pro Cys Lys Lys Phe Asp Leu Ser Tyr Asp Leu Asn Cys His Lys Leu
   500 505 510
   Cys Ser Asp Phe Gln Glu Asp Ile Val Phe Arg Phe Ser Leu Gly Trp
   515 520 525
   Ser Ser Leu Val His Arg Phe Leu Gly Ser Thr Asn Ala Gln Arg Val
   530 535 540
   Leu Leu Gly Leu Ser Glu Pro Ile Phe Gln Val Pro Arg Ser Leu Ala
   545 550 555 560
   Ser Thr Pro Thr Ala Pro Ser Asn Pro Ala Ala Pro Asp Asn Ala Ala
   565 570 575
   Gln Glu Glu Leu Met Ile Thr Leu Ile Thr Gly Leu Ala Ser Leu Thr
   580 585 590
   Ser Arg Thr Ser Met Gly Ile Ile Val Val Gly Gly Val Ile Trp Lys
   595 600 605
   Thr Val Gly Trp Lys Leu Ile Ser Val Thr Leu Ser Met Tyr Gly Ala
   610 615 620
   Leu Tyr Leu Tyr Glu Arg Leu Thr Trp Thr Thr Arg Ala Lys Glu Arg
   625 630 635 640
   Ala Phe Lys Gln Gln Phe Val Asn Tyr Ala Thr Glu Lys Leu Gln Met
   645 650 655
   Ile Val Ser Phe Thr Ser Ala Asn Cys Ser His Gln Val Gln Gln Glu
   660 665 670
   Met Ala Thr Thr Phe Ala Arg Leu Cys Gln Gln Val Asp Val Thr Gln
   675 680 685
   Lys His Leu Glu Glu Glu Ile Ala Arg Leu Ser Lys Glu Ile Asp Gln
   690 695 700
   Leu Glu Lys Ile Gln Asn Asn Ser Lys Leu Leu Arg Asn Lys Ala Val
   705 710 715 720
   Gln Leu Glu Ser Glu Leu Glu Asn Phe Ser Lys Gln Phe Leu His Pro
   725 730 735
   Ser Ser Gly Glu Ser
   740
<210> 3
   <211> 757
   <212> PRT
   <213> Homo sapiens
<400> 3
<210> 4
   <211> 757
   <212> PRT
   <213> Mus musculus
<400> 4
   Met Ser Leu Leu Phe Ser Arg Cys Asn Ser Ile Val Thr Val Lys Lys
   1 5 10 15
   Asp Lys Arg His Met Ala Glu Val Asn Ala Ser Pro Leu Lys His Phe
   20 25 30
   Val Thr Ala Lys Lys Lys Ile Asn Gly Ile Phe Glu Gln Leu Gly Ala
   35 40 45
   Tyr Ile Gln Glu Ser Ala Ser Phe Leu Glu Asp Thr His Arg Asn Thr
   50 55 60
   Glu Leu Asp Pro Val Thr Thr Glu Glu Gln Val Leu Asp Val Lys Gly
   65 70 75 80
   Tyr Leu Ser Lys Val Arg Gly Ile Ser Glu Val Leu Ala Arg Arg His
   85 90 95
   Met Lys Val Ala Phe Phe Gly Arg Thr Ser Asn Gly Lys Ser Thr Val
               100                 105                 110
   Ile Asn Ala Met Leu Trp Asp Lys Val Leu Pro Ser Gly Ile Gly His
           115                 120                 125
   Thr Thr Asn Cys Phe Leu Arg Val Gly Gly Thr Asp Gly His Glu Ala
       130                 135                 140
   Phe Leu Leu Thr Glu Gly Ser Glu Glu Lys Lys Ser Val Lys Thr Val
   145                 150                 155                 160
   Asn Gln Leu Ala His Ala Leu His Gln Asp Glu Gln Leu His Ala Gly
                   165                 170                 175
   Ser Met Val Ser Val Met Trp Pro Asn Ser Lys Cys Pro Leu Leu Lys
               180                 185                 190
   Asp Asp Leu Val Leu Met Asp Ser Pro Gly Ile Asp Val Thr Thr Glu
           195                 200                 205
   Leu Asp Ser Trp Ile Asp Lys Phe Cys Leu Asp Ala Asp Val Phe Val
       210                 215                 220
   Leu Val Ala Asn Ser Glu Ser Thr Leu Met Gln Thr Glu Lys Gln Phe
   225                 230                 235                 240
   Phe His Lys Val Ser Glu Arg Leu Ser Arg Pro Asn Ile Phe Ile Leu
                   245                 250                 255
   Asn Asn Arg Trp Asp Ala Ser Ala Ser Glu Pro Glu Tyr Met Glu Glu
               260                 265                 270
   Val Arg Arg Gln His Met Glu Arg Cys Thr Ser Phe Leu Val Asp Glu
           275                 280                 285
   Leu Gly Val Val Asp Arg Ala Gln Ala Gly Asp Arg Ile Phe Phe Val
       290                 295                 300
   Ser Ala Lys Glu Val Leu Ser Ala Arg Val Gln Lys Ala Gln Gly Met
   305                 310                 315                 320
   Pro Glu Gly Gly Gly Ala Leu Ala Glu Gly Phe Gln Val Arg Met Phe
                   325                 330                 335
   Glu Phe Gln Asn Phe Glu Arg Gln Phe Glu Glu Cys Ile Ser Gln Ser
   340 345 350
   Ala Val Lys Thr Lys Phe Glu Gln His Thr Val Arg Ala Lys Gln Ile
   355 360 365
   Ala Glu Ala Val Arg Leu Ile Met Asp Ser Leu His Ile Ala Ala Gln
   370 375 380
   Glu Gln Arg Val Tyr Cys Leu Glu Met Arg Glu Glu Arg Gln Asp Arg
   385 390 395 400
   Leu Arg Phe Ile Asp Lys Gln Leu Glu Leu Leu Ala Gln Asp Tyr Lys
   405 410 415
   Leu Arg Ile Lys Gln Ile Thr Glu Glu Val Glu Arg Gln Val Ser Thr
   420 425 430
   Ala Met Ala Glu Glu Ile Arg Arg Leu Ser Val Leu Val Asp Glu Tyr
   435 440 445
   Gln Met Asp Phe His Pro Ser Pro Val Val Leu Lys Val Tyr Lys Asn
   450 455 460
   Glu Leu His Arg His Ile Glu Glu Gly Leu Gly Arg Asn Leu Ser Asp
   465 470 475 480
   Arg Cys Ser Thr Ala Ile Ala Ser Ser Leu Gln Thr Met Gln Gln Asp
   485 490 495
   Met Ile Asp Gly Leu Lys Pro Leu Leu Pro Val Ser Met Arg Asn Gln
   500 505 510
   Ile Asp Met Leu Val Pro Arg Gln Cys Phe Ser Leu Ser Tyr Asp Leu
   515 520 525
   Asn Cys Asp Lys Leu Cys Ala Asp Phe Gln Glu Asp Ile Glu Phe His
   530 535 540
   Phe Ser Leu Gly Trp Thr Met Leu Val Asn Arg Phe Leu Gly Pro Lys
   545 550 555 560
   Asn Ser Arg Arg Ala Leu Leu Gly Tyr Ser Asp Gln Val Gln Arg Pro
   565 570 575
   Leu Pro Leu Thr Pro Ala Asn Pro Ser Met Pro Pro Leu Pro Gln Ser
   580 585 590
   Ser Leu Thr Gln Glu Glu Leu Met Val Ser Met Val Thr Gly Leu Ala
           595                 600                 605
   Ser Leu Thr Ser Arg Thr Ser Met Gly Ile Leu Val Val Gly Gly Val
       610                 615                 620
   Val Trp Lys Ala Val Gly Trp Arg Leu Ile Ala Leu Ser Phe Gly Leu
   625                 630                 635                 640
   Tyr Gly Leu Leu Tyr Val Tyr Glu Arg Leu Thr Trp Thr Thr Lys Ala
                   645                 650                 655
   Lys Glu Arg Ala Phe Lys Arg Gln Phe Val Glu Tyr Ala Ser Glu Lys
               660                 665                 670
   Leu Gln Leu Ile Ile Ser Tyr Thr Gly Ser Asn Cys Ser His Gln Val
           675                 680                 685
   Gln Gln Glu Leu Ser Gly Thr Phe Ala His Leu Cys Gln Gln Val Asp
       690                 695                 700
   Ile Thr Arg Asp Asn Leu Glu Gln Glu Ile Ala Ala Met Asn Lys Lys
   705                 710                 715                 720
   Val Glu Ala Leu Asp Ser Leu Gln Ser Arg Ala Lys Leu Leu Arg Asn
                   725                 730                 735
   Lys Ala Gly Trp Leu Asp Ser Glu Leu Asn Met Phe Thr His Gln Tyr
               740                 745                 750
   Leu Gln Pro Ser Arg
           755
<210> 5
   <211> 20
   <212> DNA
   <213> Artificial Sequence
<220>
   <223> Synthetic primer
<400> 5
   ttctggtgct tgtctcactg 20
<210> 6
   <211> 20
   <212> DNA
   <213> Artificial Sequence
<220>
   <223> Synthetic primer
<400> 6
   tatgttcggc ttcccattct 20
<210> 7
   <211> 20
   <212> DNA
   <213> Artificial Sequence
<220>
   <223> Synthetic primer
<400> 7
   caggggagat ggcacaggag 20
<210> 8
   <211> 24
   <212> DNA
   <213> Artificial Sequence
<220>
   <223> Synthetic primer
<400> 8
   cggctgtctg tcttggtgct ctcc 24
<210> 9
   <211> 21
   <212> DNA
   <213> Artificial Sequence
<220>
   <223> Synthetic primer
<400> 9
   aggggaccga tggagataaa g 21
<210> 10
   <211> 21
   <212> DNA
   <213> Artificial Sequence
<220>
   <223> Synthetic primer
<400> 10
   aagagggcac attttgcttt g 21
<210> 11
   <211> 21
   <212> DNA
   <213> Artificial Sequence
<220>
   <223> Synthetic primer
<400> 11
   acgtcaaagg gtacctgtcc a 21
<210> 12
   <211> 21
   <212> DNA
   <213> Artificial Sequence
<220>
   <223> Synthetic primer
<400> 12
   caatcccaga tggcagaact t 21

## Description

### FIELD OF THE INVENTION

The present invention relates to mitofusin-1. More specifically, the present invention relates to methods of identifying agents which are capable of binding to, or modulating the activity or expression of mitofusin-1. The present invention also relates to agents identified by these methods and the use of these agents in therapy.

### BACKGROUND TO THE INVENTION

Mitochondria are organelles present in almost every cell in the body. They play pivotal roles in a number of cellular processes, most prominently in energy metabolism. Dysfunction of mitochondria has been linked to a number of pathologies, including metabolic and cardiovascular complications, neurodegenerative disorders and cancer.

Mitochondria are, however, not single isolated organelles. Instead, they are dynamic structures capable of moving through the cell, fusing and dividing in order to adapt the respiratory capacity of the cell to its metabolic needs.

Several genes are important for mitochondrial fusion and division. Among these, mitofusin-1 and mitofusin-2 (Mfn1 and Mfn2) are responsible for mitochondrial outer membrane fusion. Both proteins are transmembrane GTPases which are localised in the mitochondrial outer membrane. The major difference between Mfn1 and Mfn2 lies in the ability of Mfn2 to connect mitochondria not just together, but also with the endoplasmic reticulum compartment in the cell (de Brito O.M. and Scorrano L. (2008) Nature 456: 605-10).

The regulation of Mfn1 and Mfn2 expression is generally orchestrated by master programs controlling mitochondrial biogenesis, for example that triggered by the activation of the peroxisome proliferator-activated receptor coactivator 1α (PGC-1α) (Soriano F.X. et al. (2006) Diabetes 55: 1783-91). Overexpression of Mfn1 and Mfn2 leads to abnormally elongated mitochondria. Conversely, down-regulation of Mfn1 and Mfn2 results in small, round-shaped mitochondria (termed "fissioned mitochondria"). Eura et al. (2006) J. Cell Sci. 119: 4913 - 4925, discloses the identification of a protein that binds mitofusin-1 and modulates its function.

Mitochondria have been shown to display highly fissioned architecture in patients with type 2 diabetes (Bach D. et al. (2003) J. Biol. Chem. 278: 17190-7). Furthermore, down-regulation of Mfn2 has also been identified in states of obesity and type 2 diabetes (Bach D. et al. (2003) J. Biol. Chem. 278: 17190-7). It has been suggested that this could explain the mitochondrial dysfunction commonly observed in diabetic patients. From these results, it was believed that down-regulation of either Mfn1 or Mfn2 could be a contributing factor in the development of diabetes.

Studying Mfn1 and Mfn2 *in vivo* was initially hampered by difficulties in the development of transgenic mouse models defective for Mfn1 or Mfn2. Germline deletion of either Mfn1 or Mfn2 causes mice to die in midgestation (Chen H. et al. (2003) J. Cell Biol. 160: 189-200). To overcome this, a conditional knockout mouse model in which Mfn2 can be deleted in a tissue-specific manner was generated. These mice were subsequently backcrossed to a C57BI/6J background to facilitate associated metabolic studies. The C57BI/6J background is the most commonly used strain for such studies, due to its ability to mimic human metabolic disease upon high-fat feeding.

Studies on the liver-specific Mfn2 deficient (Mfn2 LKO) mouse agreed with the previous work. This model demonstrated marked mitochondrial dysfunction in the liver and mice exhibited fasting hyperglycemia (Sebastian D. et al. (2012) Proc. Natl. Acad. Sci. USA 109: 5523-8). When challenged with a high fat diet, the mice were extremely sensitive to the development of insulin resistance. This confirmed that altered mitochondrial architecture could be detrimental for glucose homeostasis.

It was widely expected that analogous studies on Mfn1 deficient mice would result in a similar outcome.

### SUMMARY OF THE INVENTION

In one aspect, the present invention provides a method for identifying an agent that modulates the activity of Mfn1 comprising providing a preparation containing an Mfn1 polypeptide or polynucleotide and a candidate agent, and detecting whether said candidate agent affects activity of the Mfn1 polypeptide or polynucleotide.

The activity of Mfn1 is measured by determining the GTPase activity of Mfn1.

In another embodiment, the method comprises contacting the candidate agent with the Mfn1 polypeptide in the presence of GTP and measuring the hydrolysis of GTP to GDP.

In one embodiment, the method is for identifying an agent capable of preferentially modulating (e.g. inhibiting) the activity of Mfn1 compared to mitofusin-2 (Mfn2). The method is for identifying an agent capable of modulating the activity of Mfn1 but which does not modulate the activity of mitofusin-2 (Mfn2). The activity of Mfn2 may be measured by determining the GTPase activity of Mfn2. For instance, the agent may have a lower IC50 value for inhibition of Mfn1 than for inhibition of Mfn2. In one embodiment the agent inhibits the activity of Mfn1 but does not inhibit the activity of mitofusin-2 (Mfn2).

In another embodiment, the method is for identifying an agent that reduces the activity of Mfn1. The activity of Mfn1 may be reduced by 5%, 10%, 20%, 30%, 40%, 50%, 60%, 70%, 80%, 90% or 100% compared to the activity in the absence of the candidate agent.

In another embodiment, the method is for identifying an agent capable of treating or preventing a metabolic disease, e.g. a disease selected from the group consisting of insulin resistance, impaired glucose tolerance, impaired fasting glucose and diabetes.

In another aspect, the present invention provides a method of identifying a compound capable of modulating the interaction between Mfn1 and GTP, the method comprising the steps of:
a) providing an Mfn1 polypeptide;
b) providing a GTP molecule; and
c) detecting binding between the Mfn1 polypeptide and the GTP molecule in the presence of a candidate agent.

In another aspect, the present invention provides a method for identifying an agent that modulates the expression or processing of Mfn1 polypeptide comprising contacting cells expressing Mfn1 polypeptide with a candidate agent and monitoring an increase or decrease in expression or processing of said Mfn1 polypeptide.

In one embodiment, the method is for identifying an agent that reduces the expression or processing of Mfn1 polypeptide comprising contacting cells expressing Mfn1 polypeptide with a candidate agent and monitoring a decrease in expression or processing of said Mfn1 polypeptide. The expression of Mfn1 may be reduced by 5%, 10%, 20%, 30%, 40%, 50%, 60%, 70%, 80%, 90% or 100% compared to the expression level in the absence of the candidate agent.

In one embodiment, any of the preceding methods are for identifying an agent that prevents, modulates, reduces or ameliorates symptoms of a disease, for example a disease associated with Mfn1, or mitochondrial fusion or dysfunction. The method may be for identifying an agent that prevents, reduces or ameliorates the symptoms of a metabolic disease. The metabolic disease may be selected from the group consisting of insulin resistance, impaired glucose tolerance, impaired fasting glucose and diabetes.

In another aspect, the present invention provides a method of identifying an agent that prevents, modulates, reduces or ameliorates symptoms of a metabolic disease comprising administering an agent identified as modulating Mfn1 activity to an animal model for said metabolic disease and observing progress of the metabolic disease in said animal model.

In one embodiment, the candidate agent is identified using any of the preceding methods.

The metabolic disease may be selected from the group consisting of insulin resistance, impaired glucose tolerance, impaired fasting glucose and diabetes.

The agent of the preceding methods may be an siRNA, shRNA, miRNA, antisense RNA or small molecule.

In another aspect, the present invention provides an agent capable of modulating the activity of Mfn1, or modulating the expression or processing of Mfn1, for use in treating or preventing a metabolic disease.

In one embodiment, the agent for use in treating or preventing a metabolic disease is an agent capable of reducing the activity, expression or processing of Mfn1. The agent may be selected from the group consisting of siRNAs, shRNAs, miRNAs, antisense RNAs, polynucleotides, polypeptides or small molecules.

In another aspect, the present invention provides a vector encoding an siRNA, shRNA, miRNA, antisense RNA or a polypeptide for use in treating a metabolic disease according to the previous aspect of the invention.

The vector or agent of the invention may be for use in treating or preventing a metabolic disease selected from the group consisting of insulin resistance, impaired glucose tolerance, impaired fasting glucose and diabetes.

In another aspect, the present invention provides the use of Mfn1 polypeptide or a polynucleotide encoding the same in a method of screening for an agent that prevents or treats a metabolic disease.

In another aspect, the present invention provides the use of Mfn1 polypeptide or a polynucleotide encoding therefor as a biomarker for a metabolic disease.

In another aspect, the present invention provides the a method of diagnosing a metabolic disease or a predisposition to said disease in a subject comprising measuring expression levels of Mfn1 in a sample obtained from a subject. The metabolic disease may be selected from the group consisting of insulin resistance, impaired glucose tolerance, impaired fasting glucose and diabetes.

### DESCRIPTION OF THE DRAWINGS

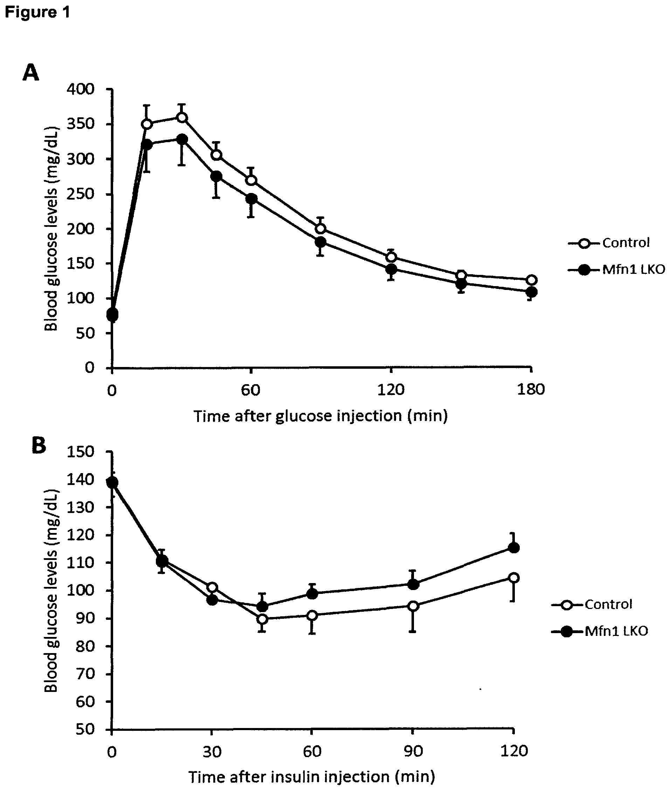

**Figure 1****. Unaffected glycemic control in Mfn1 LKO mice on chow diet.** (A) 18 week old male control or Mfn1 LKO mice were injected intraperitoneally with 2 g/kg of body weight and blood glucose levels were followed during the indicated times. (B) 20 week old male control or Mfn1 LKO mice were injected intraperitoneally with 0.3 U/kg of insulin and blood glucose levels were followed during the indicated times. All values are presented as mean +/- SEM from n=11 control mice and n=9 Mfn1 LKO mice.

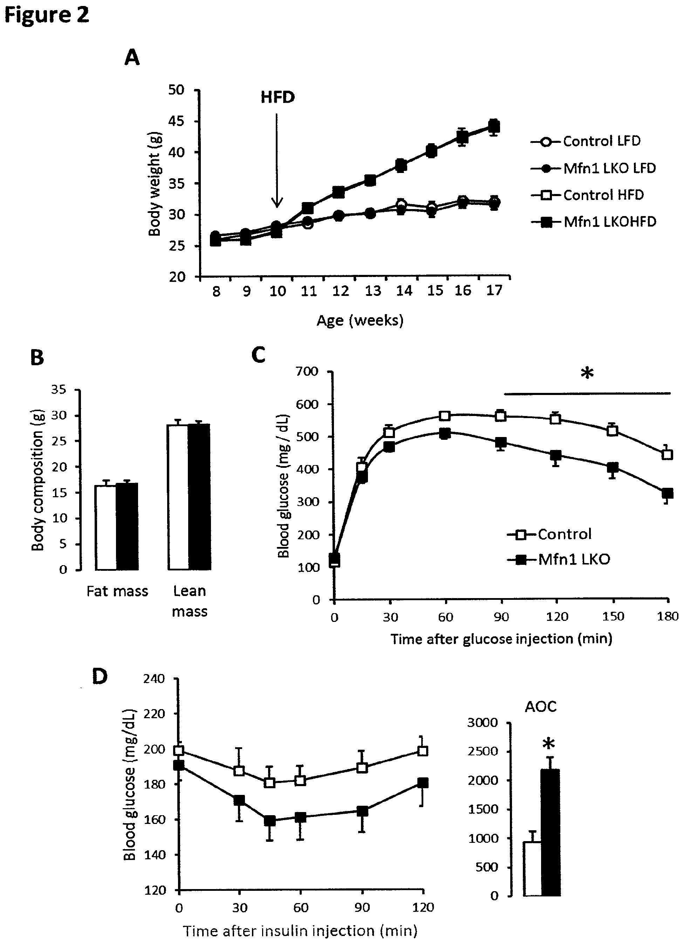

**Figure 2****. Mfn1 LKO mice display better glucose tolerance and insulin sensitivity upon high fat feeding.** (A) 10 week old control and Mfn1 LKO mice were maintained on a low fat diet (LFD) or on a high fat diet (HFD). (A) Body weight evolution. (B) Body composition after 8 weeks of high fat feeding as measured by Echo-MRI. (C) 10 weeks after the initiation of HFD, mice were injected intraperitoneally with 2 g/kg of body weight and blood glucose levels were followed during the indicated times. (D) 12 weeks after the initiation of HFD old mice were injected intraperitoneally with 0.75 U/kg of insulin and blood glucose levels were followed during the indicated times. Area over curve (AOC) quantifications are shown on the right-hand chart. All values are presented as mean +/- SEM from n=10 control mice and n=10 Mfn1 LKO mice. * indicates statistically significant difference versus respective control mice at p<0.05.

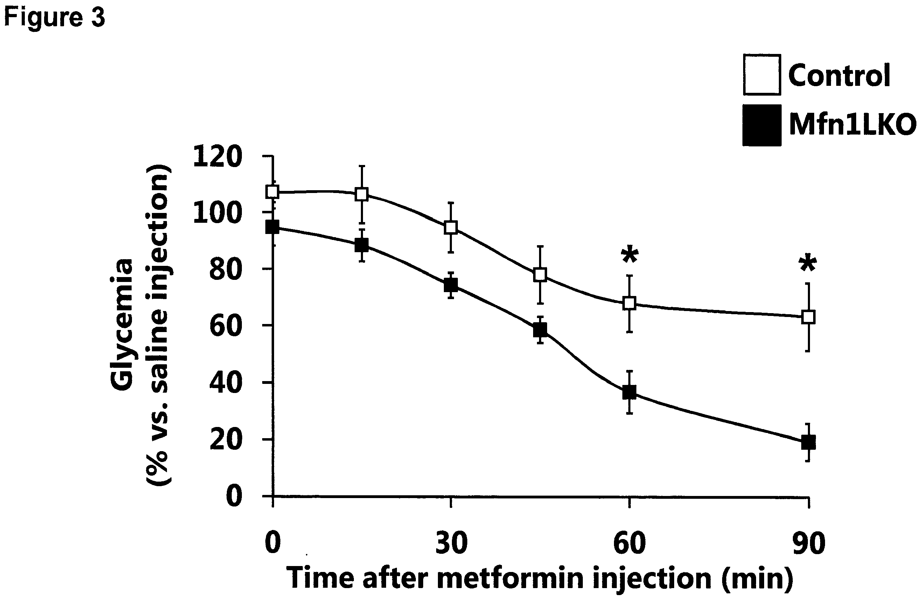

**Figure 3****. The hypoglycemic effect of metformin is enhanced in Mfn1LKO mice.**
   12 week old Control and Mfn1LKO mice were fed for 8 weeks with a high-fat diet (D12492, Research Diets Inc., USA). Then, mice were fasted overnight and injected with either saline (as vehicle) or metformin (125 mg/kg) and blood glucose levels were followed for the indicated time-points. All values are expressed as mean +/- SEM for n=10 mice per group. * indicates statistical significant difference between control and Mfn1LKO mice at P < 0.05. White squares are used for control mice values and dark squares are used for Mfn1LKO.

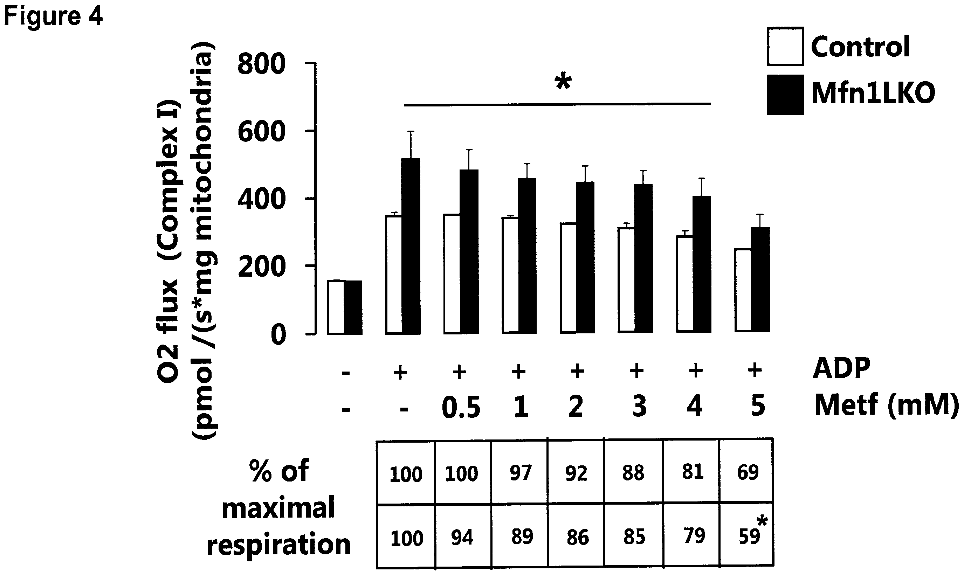

**Figure 4****. Metformin inhibition of mitochondrial respiration in WT and Mfn1LKO mice**
   Freshly extracted mitochondria from WT and Mfn1LKO mice were homogenized in respiration medium and 2 mg of tissue were used for respirometry analyses. In them, complex I activity was stimulated by the addition of malate and glutamate. Then, ADP was added to achieve maximal coupled respiration and metformin was subsequently titrated. The bar graph shows absolute respiration levels as mean +/-SEM of 4 mitochondrial preparations per genotype. * indicates statistical significant differences between genotypes at P < 0.05. The table below depicts the % of maximal respiration remaining at the different metformin doses. White bars are used for control mice and black bars for Mfn1 LKO mice.

### DETAILED DESCRIPTION OF THE INVENTION

The inventors generated a liver-specific Mfn1 deficient (Mfn1 LKO) mouse model. To achieve this, the embryonic lethality of germline Mfn1 deletion was overcome by crossing Mfn1 floxed mice (i.e. mice wherein the Mfn1 gene is flanked by two *loxP* sites) with mice in which Cre recombinase expression is controlled by the Albumin promoter. The inventors found that, while the Mfn1 LKO mice have a normal appearance and equal energy expenditure to control mice, they have a preference for fat as an energy source (Table 1). In addition, their livers display a higher respiratory capacity and respiratory complex abundance (Table 2).

Surprisingly however, when fed a regular low fat diet the Mfn1 LKO mice do not display the fasting hyperglycemia or glucose homeostasis abnormalities (Figure 1A-B) that are observed in Mfn2 LKO mice (Sebastian D. et al. (2012) Proc. Natl. Acad. Sci. USA 109: 5523-5528). Even more surprisingly, when challenged with a high-fat diet, the inventors found that the Mfn1 LKO mice gain equal weight (Figure 2A) and fat mass (Figure 2B) to control mice, but remain more glucose tolerant (Figure 2C) and insulin sensitive (Figure 2D). This significant breakthrough suggests that down-regulating Mfn1 may provide a potent strategy for combatting diabetes.

Various preferred features and embodiments of the present invention will now be described by way of non-limiting examples.

The practice of the present invention will employ, unless otherwise indicated, conventional techniques of chemistry, molecular biology, microbiology and immunology, which are within the capabilities of a person of ordinary skill in the art. Such techniques are explained in the literature. See, for example, J. Sambrook, E. F. Fritsch, and T. Maniatis (1989) Molecular Cloning: A Laboratory Manual, Second Edition, Books 1-3, Cold Spring Harbor Laboratory Press; Ausubel, F. M. et al. (1995 and periodic supplements) Current Protocols in Molecular Biology, Ch. 9, 13, and 16, John Wiley & Sons, New York, NY; B. Roe, J. Crabtree, and A. Kahn (1996) DNA Isolation and Sequencing: Essential Techniques, John Wiley & Sons; J. M. Polak and James O'D. McGee (1990) In Situ Hybridization: Principles and Practice; Oxford University Press; M. J. Gait (ed.) (1984) Oligonucleotide Synthesis: A Practical Approach, IRL Press; and, D. M. J. Lilley and J. E. Dahlberg (1992) Methods of Enzymology: DNA Structure Part A: Synthesis and Physical Analysis of DNA Methods in Enzymology, Academic Press. Each of these general texts is herein incorporated by reference.

### Mitofusins

Mitofusin-1 (Mfn1) and mitofusin-2 (Mfn2) are mammalian homologues of the *Drosophila* Fzo or yeast Fzo1 proteins. The mammalian Mfn1 and Mfn2 proteins are widely expressed in many tissues, although their expression levels vary by tissue. Both Mfn1 and Mfn2 are important for the maintenance of mitochondrial architecture.

Mfn1 and Mfn2 are anchored to the mitochondrial outer membrane by a C-terminal membrane-binding domain. Both proteins contain a cytoplasmic N-terminal GTPase domain. In addition, at least Mfn1 harbours a C-terminal heptad repeat. This region has been shown to form a dimeric anti-parallel coiled coil which mediates mitochondrial tethering (Koshiba et al. (2004) Science 305: 858-62).

Both Mfn1 and Mfn2 proteins, and their functional GTPase domains are essential for mitochondrial fusion.

In one embodiment of the present invention, Mfn1 is derived from *Homo sapiens.* For example, Mfn1 may comprise the sequence:

In another embodiment, Mfn1 is derived from *Mus musculus.* For example, Mfn1 may comprise the sequence:

In a preferred embodiment, Mfn1 comprises an amino acid sequence that has 50%, 60%, 70%, 80%, 90%, 95%, 99% or 100% identity to SEQ ID NOs: 1 or 2 and is capable of promoting mitochondrial fusion and/or integration into the mitochondrial outer membrane.

In another embodiment, the Mfn1 may be encoded by a polynucleotide comprising a nucleotide sequence which encodes the protein of SEQ ID NOs: 1 or 2, or a protein that has 50%, 60%, 70%, 80%, 90%, 95%, 99% or 100% amino acid identity to SEQ ID NOs: 1 or 2 and is capable of promoting mitochondrial fusion and/or integration into the mitochondrial outer membrane.

In one embodiment of the present invention, Mfn2 is derived from *Homo sapiens.* For example, Mfn2 may comprise the sequence:

In another embodiment, Mfn2 is derived from *Mus musculus.* For example, Mfn2 may comprise the sequence:

In a preferred embodiment, Mfn2 comprises an amino acid sequence that has 50%, 60%, 70%, 80%, 90%, 95%, 99% or 100% identity to SEQ ID NOs: 3 or 4 and is capable of mediating mitochondrial fusion.

In another embodiment, the Mfn2 may be encoded by a polynucleotide comprising a nucleotide sequence which encodes the protein of SEQ ID NOs: 3 or 4, or a protein that has 50%, 60%, 70%, 80%, 90%, 95%, 99% or 100% amino acid identity to SEQ ID NOs: 3 or 4 and is capable of mediating mitochondrial fusion.

### Mitofusin binding

The present invention includes methods for identifying agents that are capable of binding mitofusins, for example Mfn1 or Mfn2.

The method may provide either qualitative or quantitative results. The method of the invention can be used to both identify agents which are able to bind to Mfn1 or a fragment thereof and, alternatively or additionally, characterise such binding. For example, the method may allow measurement of absolute or relative binding affinity, and/or enthalpy and entropy of binding. Binding affinity may be expressed in terms of the equilibrium dissociation constant (*K*_{d}) or association (Kₐ) constant.

The candidate agents may be any agents of potential interest, for example peptides, polypeptides, nucleic acids, antibodies or small molecules. Preferably the candidate agents are compounds or mixtures of potential therapeutic interest. Preferably the candidate agents are of low toxicity for mammals, in particular humans. In some embodiments, the candidate agents may comprise nutritional agents and/or food ingredients, including naturally occuring compounds or mixtures of compounds such as plant or animal extracts.

The candidate agents may form part of a library of agents, for example a library produced by combinatorial chemistry or a phage display library. In one embodiment, the candidate agents form part of a library of plant bioactive molecules.

A number of assay techniques are known in the art for identifying binding between a candidate agent and a protein. The assay technique employed is preferably one which is amenable to automation and/or high throughput screening of candidate agents. The assay may be performed on a disposable solid support such as a microtitre plate, microbead, resin or similar.

For example, Mfn1 may be immobilised on a solid support, for example a microbead, resin, micotitre plate or array. Candidate agents may then be contacted with the immobilised Mfn1. Optionally, a wash procedure may be applied to remove weakly or non-specifically binding agents. Any agents binding to Mfn1 may then be detected and identified. To facilitate the detection of bound agents, the candidate agents may be labelled with a readily detectable marker. The marker may comprise, for example, a radio label, an enzyme label, an antibody label, a fluorescent label, a particulate (e.g. latex or gold) label or similar.

Alternatively, the above procedure may be reversed and the candidate agents may be immobilised and Mfn1 may be contacted with said immobilised agents. Optionally, a wash procedure may be applied to remove weakly or non-specifically bound Mfn1. Any agents to which Mfn1 binds may then be detected and identified. To facilitate the detection of binding, Mfn1 may be labelled with a readily detectable marker as described above.

In addition to the assays described above, other suitable assay techniques are known in the art. Examples of such techniques are radioassays, fluorescence assays, ELISA, fluorescence polarisation, fluorescence anisotropy, isothermal titration calorimetry (ITC), surface plasmon resonance (SPR) and the like. These assays may be applied to identify agents which bind to Mfn1. Indeed, platforms for the automation of many of these techniques are widely known in the art to facilitate high-throughput screening.

More than one assay techniques may be used to provide a detailed understanding of a candidate agent's binding to Mfn1. For example, assays which provide qualitative binding information may be used as a first step in the method, followed by further assays using different techniques to provide quantitative binding data.

Although techniques for identifying agents which are capable of binding to Mfn1 have been discussed above, it should be understood that these techniques are equally applicable for identifying agents which are capable of binding to Mfn2. In addition, it should be understood that these techniques will also enable identification of agents that do not bind to Mfn1 or Mfn2.

The assay techniques described above may be adapted to perform competition binding studies. For example, these techniques are equally suitable to analyse the binding of a protein to substrate or cofactor in the presence of a candidate agent. It will therefore be possible to use the above techniques to screen and identify agents that modulate the binding between a protein and its substrate or cofactor. For example, these assays will enable the detection of binding between Mfn1 or Mfn2 and GTP in the presence of a candidate agent. It will therefore be possible to identify agents that modulate Mfn1 or Mfn2 binding to GTP, for example reduce binding to GTP.

Preferably, the agents of the invention will bind to Mfn1 with high affinity. For example, the agents of the invention will bind to Mfn1 with a Kd of less than 100 µM, more preferably less than 10 µM, for example less than 1 µM, less than 100 nM or less than 10 nM.

The agents of the present invention typically bind preferentially to Mfn1 compared to Mfn2. In other words, the agents are typically selective for Mfn1 over Mfn2, e.g. the agents bind with higher affinity to Mfn1 than to Mfn2 under the same conditions. Binding affinity may be measured using standard techniques known in the art, e.g. surface plasmon resonance, ELISA and so on (for instance as described above), and may be quantified in terms of either association (Kₐ) or dissociation (K_{d}) constants.

In a preferred embodiment, the agent binds to Mfn1 and Mfn2 with an affinity ratio of at least 2:1 (e.g. Kₐ (Mfn1)/ Kₐ (Mfn2) ≥ 2). In further embodiments, the agent may have an affinity ratio for Mfn1/Mfn2 of at least 5:1, at least 10:1, at least 100:1, at least 1000:1 or at least 10,000:1. For instance, the agent may bind to Mfn1 with a K_{d} of less than 100 µM, preferably less than 1 µM, more preferably less than 100 nM, most preferably less than 10 nM. The agent may bind to Mfn2 with a K_{d} of greater than 10 nM, preferably greater than 100 nM, more preferably greater than 1 µM, most preferably greater than 100 µM. In one embodiment the agent does not bind to Mfn2 (e.g. the agent shows negligible or substantially no binding to Mfn2 under standard assay conditions).

### Mitofusin activity

The present invention also includes methods for identifying agents that are capable of modulating the activity of mitofusins, for example Mfn1 or Mfn2. The activity of Mfn1 or Mfn2 may be analysed directly, for example by analysing the enzymatic activity of the Mfn1 or Mfn2 GTPase. Alternatively or additionally, the activity of Mfn1 or Mfn2 may be analysed indirectly, for example by analysing the fusion of mitochondria within a cell.

The ability of a candidate agent to reduce the activity of a protein, for example an enzyme such as a GTPase, may be expressed in terms of an IC50 value. The IC50 is the concentration of an agent that is required to give rise to a 50% reduction in the activity of the protein (e.g. a 50% reduction in enzymatic activity). The calculation of IC50 values is well known in the art. Preferably, the agents of the invention have an IC50 value for inhibition of Mfn1 activity (e.g. GTPase activity) of less than 100 µM, more preferably less than 10 µM, for example less than 1 µM, less than 100 nM or less than 10 nM.

The agents of the present invention typically preferentially inhibit the activity of Mfn1 compared to Mfn2. In other words, the agents typically selectively inhibit Mfn1, e.g. the agents have a lower IC50 value for inhibition of Mfn1 activity compared to Mfn2 under the same conditions. In a preferred embodiment, the agent inhibits Mfn2 and Mfn1 with an IC50 ratio of at least 2:1 (e.g. IC50 (Mfn2)/ IC50 (Mfn1) ≥ 2). In further embodiments, the agent may have an IC50 ratio for Mfn2/Mfn1 of at least 5:1, at least 10:1, at least 100:1, at least 1000:1 or at least 10,000:1. For instance, the agent may have an IC50 for inhibition of Mfn1 of less than 100 µM, preferably less than 1 µM, more preferably less than 100 nM, most preferably less than 10 nM. The agent may have an IC50 for inhibition of Mfn2 of greater than 10 nM, preferably greater than 100 nM, more preferably greater than 1 µM, most preferably greater than 100 µM. In one embodiment the agent does not inhibit the activity of Mfn2 (e.g. the agent shows negligible or substantially no inhibition of the activity of Mfn2 under standard assay conditions).

### GTPase assays

A number of techniques are known in the art for measuring GTPase activity. Such GTPase assays may be based on the detection of GTP to GDP conversion by the GTPase which occurs with concomitant release of inorganic phosphate. These techniques may be applied to a GTPase, for example Mfn1 or Mfn2, that has been isolated from a cell. The Mfn1 or Mfn2 may have been expressed using recombinant techniques. Preferably, the Mfn1 or Mfn2 has been purified. Methods for the expression and purification of Mfn1 and Mfn2 are known in the art.

In one embodiment, GTPase activity may be determined by monitoring GTP to GDP conversion using [α-³²P]-labelled GTP. Briefly, thin layer chromatography separation of GTP and GDP may be performed on GTPase reactions at various time-points. Relative levels of GTP and GDP at each time point may then be quantified by phosphorimager analysis. Suitable [α-³²P]-based GTPase assays are described in Ishihara N. et al. (2004) J. Cell Sci. 117: 6535-46 (applied to Mfn1) and Warnock D.E. et al. (1996) J. Biol. Chem. 271: 22310-4.

In another embodiment, GTPase activity may be determined using an assay to detect the release of inorganic phosphate (Pᵢ) which is produced upon GTP hydrolysis. For example, malachite green colourimetric assays are well known in the art for detecting the release of inorganic phosphate during enzyme reactions. Briefly, these assays are based on the principle that the malachite green detection agent undergoes a colour change upon binding inorganic phosphate. The colour change may be measured by spectrophotometric detection which allows quantification of the amount of inorganic phosphate released over time. Suitable commercial malachite assays are available, for example the GTPase assay from Innova Biosciences. Such colourimetric assays are well-suited to high-throughput screening.

### Mitochondrial fusion assays

The effect of candidate agents on mitochondrial fusion may also be analysed directly within a cell. Methods for analysing mitochondrial fusion are well known in the art. For example, mitochondria may be visualised in cells using time-lapse confocal microscopy. This technique is described in Chen *et al.* (Chen H. et al. (2003) J. Cell Biol. 160: 189-200) to study the effect of Mfn1 and Mfn2 knockouts on mitochondrial dynamics and fusion.

### Mitofusin expression

Methods for analysing the expression of Mfn1 or Mfn2 may be employed in the present invention to screen the effect of a candidate agent on protein levels. In addition, expression levels of a protein, for example Mfn1, Mfn2 or other biomarker, may be analysed in a sample from a subject as part of a method of diagnosis. The expression levels of the protein, may correlate with a disease or pre-disposition to a disease.

A number of techniques are known in the art for determining the expression level of a protein. These techniques may be applied to test the effect of candidate agents on the expression level of Mfn1 and/or Mfn2. The technique employed is preferably one which is amenable to automation and/or high throughput screening of candidate agents.

For example, screens may be carried out using cells harbouring nucleic acids encoding Mfn1 and/or Mfn2 operably linked to a reporter moiety. The reporter moiety may be operably linked to endogenous Mfn1- and/or Mfn2-encoding genes. Alternatively, exogenous copies of Mfn1 and/or Mfn2 operably linked to a reporter moiety may be inserted into a cell. In this embodiment, the cell may be engineered to be deficient for natural Mfn1 and/or Mfn2 expression. The reporter moieties linked to Mfn1 and Mfn2 may be different and distinguishable from one another. Suitable reporter moieties include fluorescent labels, for example fluorescent proteins such as green, yellow, cherry, cyan or orange fluorescent proteins.

By "operably linked" it is to be understood that the components described are in a relationship permitting them to function in their intended manner.

Such cells may be contacted with candidate agents and the level of expression of Mfn1 and/or Mfn2 may be monitored by analysing the level of reporter moiety expression in the cell. Fluorescent reporter moieties may be analysed by a number of techniques known in the art, for example flow cytometry, fluorescence-activated cell sorting (FACS) and fluorescence microscopy. Expression of Mfn1 or Mfn2 may be analysed separately or simultaneously within the same cell. Expression levels of Mfn1 and/or Mfn2 may be compared before and after contact with the candidate agent. Alternatively, expression levels of Mfn1 and/or Mfn2 may be compared between cells contacted with a candidate agent and control cells. If the effect on expression of both Mfn1 and Mfn2 are determined, the effect of the candidate agent may be expressed as the ratio of Mfn1 and Mfn2 expression

Other methods may be used for analysing the expression of proteins, for example Mfn1 and Mfn2. Protein expression may be analysed directly. For example, expression may be quantitatively analysed using methods such as SDS-PAGE analysis with visualisation by Coomassie or silver staining. Alternatively expression may be quantitatively analysed using Western blotting or enzyme-linked immunosorbent assays (ELISA) with antibody probes which bind the protein product. Mfn1 and/or Mfn2 labelled with reporter moieties, as described above, may also be used in these methods. Alternatively, protein expression may be analysed indirectly, for example by studying the amount of mRNA corresponding to the protein, for example Mfn1 and/or Mfn2, that is transcribed in a cell. This can be achieved using methods such as quantitative reverse transcription PCR and Northern blotting.

### siRNAs, shRNAs, miRNAs and antisense DNAs/RNAs

Expression of mitofusins may be modulated using post-transcriptional gene silencing (PTGS). Post-transcriptional gene silencing mediated by double-stranded RNA (dsRNA) is a conserved cellular defence mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi) (Ralph et al. (2005) Nat. Medicine 11: 429-33). The mechanism of RNAi involves the processing of long dsRNAs into duplexes of about 21-25 nucleotide (nt) RNAs. These products are called small interfering or silencing RNAs (siRNAs) which are the sequence-specific mediators of mRNA degradation. In differentiated mammalian cells, dsRNA >30 bp has been found to activate the interferon response leading to shut-down of protein synthesis and non-specific mRNA degradation (Stark et al. (1998) Ann. Rev. Biochem. 67: 227-64). However, this response can be bypassed by using 21 nt siRNA duplexes (Elbashir et al. (2001) EMBO J. 20: 6877-88; Hutvagner et al. (2001) Science 293: 834-8) allowing gene function to be analysed in cultured mammalian cells.

shRNAs consist of short inverted RNA repeats separated by a small loop sequence. These are rapidly processed by the cellular machinery into 19-22 nt siRNAs, thereby suppressing the target gene expression.

Micro-RNAs (miRNAs) are small (22-25 nucleotides in length) noncoding RNAs that can effectively reduce the translation of target mRNAs by binding to their 3' untranslated region (UTR). Micro-RNAs are a very large group of small RNAs produced naturally in organisms, at least some of which regulate the expression of target genes. Founding members of the micro-RNA family are *let-7* and *lin-4.* The *let-7* gene encodes a small, highly conserved RNA species that regulates the expression of endogenous protein-coding genes during worm development. The active RNA species is transcribed initially as an ∼70 nt precursor, which is post-transcriptionally processed into a mature ∼21 nt form. Both *let-7* and *lin-4* are transcribed as hairpin RNA precursors which are processed to their mature forms by Dicer enzyme.

The antisense concept is to selectively bind short, possibly modified, DNA or RNA molecules to messenger RNA in cells and prevent the synthesis of the encoded protein.

Methods for the design of siRNAs, shRNAs, miRNAs and anti-sense DNAs/RNAs to modulate the expression of a target protein, and methods for the delivery of these agents to a cell of interest are well known in the art. Furthermore, methods for specifically modulating (e.g. reducing) expression of a protein in a certain cell type within an organism, for example through the use of tissue-specific promoters are well known in the art.

### Metabolic diseases

Agents of the invention may be used in the treatment or prevention of a metabolic disease. The metabolic disease may be diabetes mellitus, insulin resistance, impaired glucose tolerance or impaired fasting glucose. Preferably the metabolic disease is diabetes mellitus. The diabetes mellitus may be type 1 or type 2 diabetes, preferably type 2 diabetes.

Diabetes mellitus is a metabolic condition characterised primarily by high blood glucose levels that result from the body's inability to make or use insulin. Hyperglycemia can lead to numerous clinical complications including neuronal damage, blindness, limb amputations, kidney impairments, retinopathy, atherosclerosis, arteriosclerosis, diabetes-derived gangrene, heart attacks and strokes.

The most common types of diabetes are insulin-dependent diabetes (type-1 diabetes), and type 2 diabetes, which is by far the most abundant type. The increase in type 2 diabetes is mainly driven by increasing obesity rates. Today, more than 1.1 billion people are estimated to be overweight, of which around 320 million are obese.

Type 1 diabetes is an autoimmune disease which leads to destruction of insulin-producing pancreatic beta cells. Type 2 diabetes is a state where either insulin is secreted in an inadequate amount (insulin deficiency) or the effect of insulin on suppressing blood sugar levels is weakened (insulin tolerance or resistance).

The pathophysiology of the development of type 2 diabetes is complex and multifactorial. Obesity, sedentary lifestyle and/or increased age may lead to insulin resistance and to increased circulating insulin concentrations over time. At some point, a loss of control of blood glucose begins to emerge, resulting in impaired glucose tolerance or impaired fasting glucose. Ultimately type 2 diabetes may result. Therefore insulin resistance, impaired glucose tolerance and impaired fasting glucose refer to metabolic states intermediate between normal glucose homeostasis and diabetes.

### Animal models of metabolic disease

A number of animal models of metabolic diseases, for example diabetes, insulin resistance and impaired glucose tolerance, are known in the art. As discussed above, the C57BI/6J mouse model is commonly used for animal-based studies, due to its ability to mimic human metabolic disease upon high-fat feeding.

Models of type 1 diabetes may be developed by chemical ablation of beta cells or breeding of rodents that develop autoimmune disease. Chemical ablation of rodents or higher animals may be achieved, for example, using STZ or alloxan. Autoimmune models of type 1 diabetes include NOD mice, BB rats and LEW.1AR1/-iddm rats. In addition, AKITA mice, which have genetically-induced insulin dependent diabetes, may be used as models of type 1 diabetes. Higher organism models, for example pigs and Cynomolgus monkeys, may be used as models of type 1 diabetes following pancreatectomy or chemical ablation of beta cells.

Models of type 2 diabetes generally also model insulin resistance. Many type 2 diabetes models are obese, for example Lep^{ob/ob} mice, Lep^{db/db} mice, Zucker fatty and Zucker diabetic fatty rats, KK mice, OLETF rats, New Zealand obese mice and the like. Type 2 diabetes models may also be created by high fat feeding, for example of C57BI/6J mice. Non-obese models of type 2 diabetes include Goto-Kakizaki rats and hIAPP mice. In addition, cat, dog, pig and Old-World non-human primate models of type 2 diabetes are known in the art. These model systems may be used in the methods of the present invention.

Suitable animal models known in the art are reviewed in detail in King *et al.* (King A.J. et al. (2012) Br. J. Pharmacol. 166: 877-94). In addition, the Mfn1 LKO and Mfn2 LKO mice discussed above may also be used as animal models of metabolic disease.

### Introduction of polypeptides and polynucleotides into cells

An agent for use in the invention may be a polypeptide or a polynucleotide. Polynucleotides and polypeptides may also need to be introduced into cells as part of the methods or screening assays of the present invention.

Where the invention makes use of a polypeptide, the polypeptides may be administered directly (e.g. the polypeptide itself may be administered), or by introducing nucleic acid sequences encoding the polypeptide into cells under conditions that allow for expression of the polypeptide in a cell of interest. Nucleic acids may be introduced into cells using vectors.

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid, or facilitating the expression of the protein encoded by a segment of nucleic acid. Vectors may be non-viral or viral. Examples of vectors used in recombinant nucleic acid techniques include, but are not limited to, plasmids, chromosomes, artificial chromosomes and viruses. The vector may also be, for example, a naked nucleic acid (e.g. DNA). In its simplest form, the vector may itself be a nucleotide of interest.

The vectors used in the invention may be, for example, plasmid or virus vectors and may include a promoter for the expression of a polynucleotide and optionally a regulator of the promoter.

Vectors comprising polynucleotides used in the invention may be introduced into cells using a variety of techniques known in the art, such as transformation and transduction. Several techniques are known in the art, for example infection with recombinant viral vectors, such as retroviral, lentiviral, adenoviral, adeno-associated viral, baculoviral and herpes simplex viral vectors; direct injection of nucleic acids and biolistic transformation.

Non-viral delivery systems include but are not limited to DNA transfection methods. Here, transfection includes a process using a non-viral vector to deliver a gene to a target cell.

Transfer of the polypeptide or polynucleotide may be performed by any of the methods known in the art which may physically or chemically permeabilise the cell membrane. Cell-penetrating peptides may also be used to transfer a polypeptide into a cell.

Non-viral delivery systems include, but are not limited to, DNA transfection methods and calcium phosphate precipitation. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology 1996 14; 556) and combinations thereof.

In addition, the invention may employ gene targeting protocols, for example the delivery of DNA-modifying agents.

The vector may be an expression vector. Expression vectors as described herein comprise regions of nucleic acid containing sequences capable of being transcribed. Thus, sequences encoding mRNA, tRNA and rRNA are included within this definition.

Expression vectors preferably comprise a polynucleotide for use in the invention operably linked to a control sequence that is capable of providing for the expression of the coding sequence by the host cell. A regulatory sequence "operably linked" to a coding sequence is ligated in such a way that expression of the coding sequence is achieved under conditions compatible with the control sequence. The control sequence may be modified, for example by the addition of further transcriptional regulatory elements to make the level of transcription directed by the control sequence more responsive to transcriptional modulators.

### Polynucleotides

Polynucleotides of the invention may comprise DNA or RNA. They may be single-stranded or double-stranded. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The polynucleotides may be modified by any method available in the art. Such modifications may be carried out in order to enhance the *in vivo* activity or lifespan of the polynucleotides of the invention.

Polynucleotides, such as DNA polynucleotides, may be produced recombinantly, synthetically or by any means available to those of skill in the art. They may also be cloned by standard techniques.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA, for example mRNA or cDNA obtained from an animal or human cell, performing a polymerase chain reaction under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

### Proteins

As used herein, the term "protein" includes single chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds.

### Variants, derivatives, analogues, homologues and fragments

In addition to the specific proteins and nucleotides mentioned herein, the present invention also encompasses variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring protein or nucleotide.

The term "derivative" as used herein, in relation to proteins or polypeptides of the present invention, includes any substitution of, variation of, modification of, replacement of, deletion of and/or addition of one (or more) amino acid residues from or to the sequence, providing that the resultant protein or polypeptide retains at least one of its endogenous functions.

The term "analogue" as used herein, in relation to polypeptides or polynucleotides, includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions, provided that the modified sequence retains the required activity or ability. Amino acid substitutions may include the use of non-naturally occurring analogues.

Proteins used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" means an entity having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95% or 97% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95% or 97% or 99% identical to the subject sequence. Although homology can also be considered in terms of similarity, in the context of the present invention it is preferred to express homology in terms of sequence identity.

Homology comparisons can be conducted by eye, or more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Research 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174(2):247-50; FEMS Microbiol. Lett. (1999) 177(1):187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate percentage homology, preferably percentage sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

All variants, fragments or homologues of the mitofusins (e.g. Mfn1 or Mfn2) of the invention will retain the ability to bind mediate mitochondrial fusion.

### Codon optimisation

The polynucleotides used in the present invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells, as well as for a variety of other organisms.

### Antibodies

Antibodies, as used herein, refers to complete antibodies or antibody fragments capable of binding to a selected target, and including Fv, ScFv, F(ab') and F(ab')₂, monoclonal and polyclonal antibodies, engineered antibodies including chimeric, CDR-grafted and humanised antibodies, and artificially selected antibodies produced using phage display or alternative techniques.

In addition, alternatives to classical antibodies may also be used in the invention, for example "avibodies", "avimers", "anticalins", "nanobodies" and "DARPins".

Methods for the production of antibodies are known by those skilled in the art. Alternatively, antibodies may be derived from commercial sources.

If polyclonal antibodies are desired, a selected mammal (e.g. mouse, rabbit, goat or horse) may be immunised. Serum from the immunised animal may be collected and treated according to known procedures. If the serum contains polyclonal antibodies to other antigens, the polyclonal antibodies may be purified by immunoaffinity chromatography. Techniques for producing and processing polyclonal antisera are known in the art.

Monoclonal antibodies directed against antigens (e.g. proteins) used in the invention can also be readily produced by those skilled in the art. The general methodology for making monoclonal antibodies by hybridomas is well known. Immortal antibody-producing cell lines can be created by cell fusion and also by other techniques such as direct transformation of B-lymphocytes with oncogenic DNA or transfection with Epstein-Barr virus. Panels of monoclonal antibodies produced against antigens can be screened for various properties, for example for isotype and epitope affinity.

An alternative technique involves screening phage display libraries where, for example, the phage express scFv fragments on the surface of their coat with a large variety of complementarity determining regions (CDRs). This technique is well known in the art.

Antibodies, both monoclonal and polyclonal, which are directed against antigens are particularly useful in diagnosis, and those which are neutralising are useful in passive immunotherapy. Monoclonal antibodies in particular may be used to raise anti-idiotype antibodies. Anti-idiotype antibodies are immunoglobulins which carry an "internal image" of the antigen of the infectious agent against which protection is desired.

Techniques for raising anti-idiotype antibodies are known in the art. These anti-idiotype antibodies may also be useful for treatment, as well as for an elucidation of the immunogenic regions of antigens.

### Method of treatment

It is to be appreciated that all references herein to treatment include curative, palliative and prophylactic treatment. The treatment of mammals, particularly humans, is preferred. Both human and veterinary treatments are within the scope of the present invention.

### Administration

Even though the agents for use in the present invention can be administered alone, they will generally be administered in admixture with a pharmaceutical carrier, excipient or diluent, particularly for human therapy. In some embodiments, the agent is a nutritional agent, food additive or food ingredient, and may thus be formulated in a suitable food composition. Thus the agent may be administered, for example, in the form of a food product, drink, pet food product, food supplement, nutraceutical, or nutritional formula.

### Dosage

A person of ordinary skill in the art can easily determine an appropriate dose of one of the agents of the invention to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can of course be individual instances where higher or lower dosage ranges are merited, and such are within the scope of this invention.

### Biomarkers and methods of diagnosis

### Sample

A "sample" in the context of diagnostic and prognostic methods is understood within the scope of the invention to refer to a sample which is derived from a subject. The biological sample may be obtained directly from the subject or may be derived from cultured cells obtained from said subject.

A "sample" in the context of screening assays is understood within the scope of the invention to refer to a suitable cell, group of cells, animal model or human. These samples do not have to be derived from a subject. A sample in this context can be a group of cells from a cell line.

### Subject

A "subject" refers to either a human or non-human animal. Examples of non-human animals include vertebrates, for example mammals, such as non-human primates (particularly higher primates), dogs, rodents (e.g. mice, rats or guinea pigs), pigs and cats. In a preferred embodiment, the subject is a human.

### EXAMPLES

### EXAMPLE 1: General methodologies:

*Materials.* Unless otherwise stated, all chemicals, including PJ34, were from Sigma-Aldrich.

*Animal experiments.* Male *Mfn1* floxed mice on a pure C57B1/6J background were created by Prof. Antonio Zorzano at IRB Barcelona (Spain). The mice were then crossed with mice expressing the Cre-recombinase enzyme in order to generate mice with a liver-specific deletion of the Mfn1 gene (Mfn1-LKO). Floxed animals were used as control. Mice were housed with *ad libitum* access to water and standard rodent chow (D12450J, Research diets Inc., New Brunswick, NJ, USA) or to a high calorie, high fat diet (60 kcal of fat, *Research Diets,* New Brunswick, NJ, USA), and were kept under a 12h dark-light cycle. All animal experiments were carried out according to local national and EU ethical guidelines. To monitor body weight, mice were weighed and the food consumption was measured each week on the same day. In all studies animals were killed (at 14:00) either after 6h of fasting (starting at 8:00), and tissues were collected and processed as specified below. Oral glucose tolerance test and intraperitoneal insulin tolerance test were determined as described before (Lagouge et al, 2006). Plasma insulin and was determined in heparinized plasma samples using specific ELISA kits *(Mercodia).* Blood samples were collected in heparinized tubes and plasma was isolated after centrifugation. We measured O₂ consumption, CO₂ production, and spontaneous locomotor activity in an open -circuit indirect calorimetry system *(Sabre systems,* Las Vegas, NV, USA) over 24-48h as described (Lagouge et al, 2006). Energy expenditure was obtained by using an energy equivalent of 20. 1 J/ml O2. The respiratory quotient was the ratio of CO₂ production over O₂ consumption. During actimetry, beamline crossings were summarized each 15 minutes. The sum of beamline crosses for each 15 min period was plotted against time and AUC was calculated for each mouse that was average in each experimental cohort.

*mRNA analysis:* Total RNA was prepared using TRIzol *(Invitrogen)* according to the manufacturer's instructions. RNA was treated with DNase, and 2 mg of RNA was used for reverse transcription (RT). cDNA was purified on QIAquick PCR cleanup columns *(Qiagen,* Valencia, CA, USA). 50X diluted cDNA was used for RT-quantitative PCR (RT-qPCR) reactions (Lagouge et al, 2006). The RT-qPCR reactions were performed using the Light-Cycler system *(Roche Applied Science)* and a qPCR Supermix *(Qiagen)* with the following primers: beta-2-microglobulin (housekeeping; F: TTCTGGTGCTTGTCTCACTG (SEQ ID NO:5); R: TATGTTCGGCTTCCCATTCT (SEQ ID NO:6)); cyclophylin (housekeeping: F: CAGGGGAGATGGCACAGGAG (SEQ ID NO:7); R: CGGCTGTCTGTCTTGGTGCTCTCC (SEQ ID NO:8)); Mfn1 (F: AGGGGACCGATGGAGATAAAG (SEQ ID NO:9); R: AAGAGGGCACATTTTGCTTTG (SEQ ID N0:10)); Mfn2 (F: ACGTCAAAGGGTACCTGTCCA (SEQ ID NO:11); R: CAATCCCAGATGGCAGAACTT (SEQ ID NO:12)). Mfn1 and Mfn2 expression estimations were corrected by the two housekeeping genes.

*Mitochondrial isolation, SDS-PAGE, Western blotting.* Approximately 0.5 mg of liver were used to isolate mitochondria as previously described (Frezza et al, 2007). The final mitochondrial pellet was resuspended in lysis buffer (50 mM Tris, 100 mM KCl, EDTA 1 mM, NP40 1%, nicotinamide 5mM, Na-butyrate 1mM, protease inhibitors pH7.4). Protein concentration was measured using a BCA protein assay kit (Thermo Scientific, Rockford, IL, USA), and sample dilutions were adjusted to yield equal protein concentration Proteins were separated by SDS -PAGE and transferred onto nitrocellulose membranes as previously described (Canto et al, 2004).

*Antibodies used for western blot applications.* Mfn1, porin and OXPHOS cocktail antibodies were purchased from Abcam (Cambridge, UK), OPA-1 antibody was purchased from BD Biosciences (San Francisco, USA). Peroxidase-conjugated secondary mouse and rabbit antibodies were purchased from Sigma-Aldrich.

*Mitochondrial respiration:* Mitochondrial function was assessed using respirometry (Oroboros Oxygraph-2k; Oroboros Instruments, Innsbruck, Austria), as previously described (Holmstrom et al, 2012). A piece of freshly extracted liver (approx. 50 mg) was weighted and placed in a relaxing solution (2.8 mm Ca2K2EGTA, 7.2 mm K2EGTA, 5.8 mm ATP, 6.6 mm MgCI2, 20 mm taurine, 15 mm sodium phosphocreatine, 20 mm imidazole, 0.5 mm dithiothreitol, and 50 mm MES, pH 7.1), and at the end of the dissection procedures, the tissue was gently homogenized in 200 microliters of ice-cold respirometry medium (0.5 mm EGTA, 3 mm MgCI2, 60 mm potassium lactobionate, 20 mm taurine, 10 mm KH2PO4, 20 mm HEPES, 110 mm sucrose, and 0.1% (w/v) bovine serum albumin, pH 7.1) using an eppendorf motor pestle. The volume of respirometry medium was then adjusted to obtain a concentration of 0.1 mg of tissue/microliter. Then, 2 mg of homogenate was loaded into the respiration chamber. All measurements were made in duplicate. Basal O2 flux from complex I (Leak) of the respiratory chain was measured by adding the substrates malate (final concentration 2 mM), glutamate (20 mM), and pyruvate (10 mM). Oxidative phosphorylation was quantified by the addition of ADP (5 mM), followed by the complex II substrate succinate (10 mM), for convergent electron flow through Complexes I + II. Then the protonophore carbonylcyanide-4-(trifluoromethoxy)-phenylhydrazone (0.2 µM) was titrated to achieve maximum flux trough the electron transfer system. Finally, electron transport through complexes I and III was inhibited by the sequential addition of rotenone (0.1 µM) and antimycin A (2.5 µM), respectively.

*Statistics.* For the statistical analysis in the animal studies, all data was verified for normal distribution. To assess significance we performed Student's t-test for independent samples. The statistical software used was GraphPad Prism 5 (GraphPad Software, Inc.) and all p-values <0,05 were considered significant.

**EXAMPLE 2. Liver-specific deletion of Mfn1 (Mfn1-LKO) does not alter global appearance, food intake, physical activity or energy expenditure, but enhances whole body lipid oxidation rates.** (Table 1) 16 week old Mfn1LKO and control mice were subjected to Echo-MRI analysis to analyze body composition and immediately thereafter, submitted to CLAMS analysis to evaluate daily activity and food intake. At 24 weeks of age mice were sacrificed and total mRNA was extracted from mouse livers to analyze Mfn1 and Mfn2 expression through RT-qPCR.

**EXAMPLE 3. Livers from Mfn1LKO mice display higher respiratory capactity.** (Table 2) Control and Mfn1LKO mice were sacrificed at 24 weeks of age. (A) Total liver homogenates or were used for respirometry assays as described in Example 1. All values are presented as mean +/- SEM from n=11 for control mice and n=9 for Mfn1 LKO mice.

**EXAMPLE 4. Unaffected glycemic control in Mfn1LKO mice on chow diet.** (Figure 1) (A) 18-week old male control or Mfn1LKO Mice were injected intraperitoneally with 2 g/kg of body weight and blood glucose levels were followed during the indicated times. (B) 20-week old male contorl or Mfn1-LKO mice were injected intraperitoneally with 0.3 U/Kg of insulin and blood glucose levels were followed during the indicated times. All values are presented as mean +/- SEM from n=11 control mice and n=9 Mfn1-LKO.

**EXAMPLE 5. Mfn1LKO mice display better glucose tolerance and insulin sensitivity upon high-fat feeding.** (Figure 2) (A) 10 week old control and Mfn1LKO mice were maintained on low fat diet (LFD) or on high fat diet (HFD). (A) Body weight evolution. (B) Body composition after 8 weeks of high-fat feeding as measured by Echo-MRI. (C) 10 weeks after the initiation of HFD, mice were injected intraperitoneally with 2 g/kg of body weight and blood glucose levels were followed during the indicated times. (D) 12-weeks after the initiation of HFD old mice were injected intraperitoneally with 0.75 U/Kg of insulin and blood glucose levels were followed during the indicated times. Area over curve (AOC) quantifications are shown on the right. All values are presented as mean +/- SEM from n=10 control mice and n=10 Mfn1LKO. * indicates statistical significant difference vs. respective control mice at p<0.05.

### EXAMPLE 6 - Hypoglycemic effect of metformin is enhanced in Mfn1LKO mice

Metformin is an oral antidiabetic drug in the biguanide class. It is the first-line drug of choice for the treatment of type 2 diabetes, in particular, in overweight and obese individuals and those with normal kidney function. It is also used in diseases where insulin resistance may be an important factor. Metformin works by suppressing glucose production by the liver. Metformin is sometimes prescribed in combination with insulin or other medications.

As seen in Figure 3, the hypoglycemic effect of metformin is enhanced in Mfn1LKO mice. 12 week old Control and Mfn1LKO mice were fed for 8 weeks with a high-fat diet (D12492, Research Diets Inc., USA). Then, mice were fasted overnight and injected with either saline (as vehicle) or metformin (125 mg/kg) and blood glucose levels were followed for the indicated time-points. All values are expressed as mean +/- SEM for n=10 mice per group. * indicates statistical significant difference between control and Mfn1LKO mice at P < 0.05.

### EXAMPLE 7- Enhanced AMPK activation upon metformin injection in Mfn1LKO mice

12 week old Control and Mfn1LKO mice were fed for 8 weeks with a high-fat diet (D12492, Research Diets Inc., USA). Then, mice were fasted overnight and injected with either saline (as vehicle) or metformin (125 mg/kg). Mice were sacrificed 90 minutes later and livers were immediately extracted and frozen. Protein extracts were obtained and used for western blot analyses to test the markers of AMPK activation: CRTC2, elF2alpha and GAPDH. Compared to the control mice, the Mfn1 LKO mice showed an enhanced AMPK activation as demonstrated by the measured markers of AMPK activation.

### EXAMPLE 8 - Metformin inhibition of mitochrondrial respiration in wild type and Mfn1LKO mice

Methods to measure mitochondrial respiration are described above in Example 1. Freshly extracted mitochondria from WT and Mfn1 LKO mice were resuspended in respiration medium and 100 µg of protein were used for respirometry analyses. Complex I activity was stimulated by the addition of malate and glutamate. Then, ADP was added to achieve maximal coupled respiration and metformin was subsequently titrated. Figure 4 bar graph shows absolute respiration levels as mean +/-SEM of 4 mitochondrial preparations per genotype. * indicates statistical significant differences between WT and Mfn1LKO at P < 0.05. The table below depicts the percent of maximal respiration remaining at the different metformin doses.

In general, metformin action is still not well-understood, even though inhibition of Complex I is believed to be a major contributor (Metformin: From Mechanisms of Action to Therapies, Foretz et al., 2014, Cell Metabolism; DOI: 10.1016/j.cmet.2014.09.018) As demonstrated in this example, mitochondrial Complex I respiration is inhibited more in the Mfn1LKO mice than in the control mice after administration of metformin.

**Table 1: Global appearance and energy expenditure parameters in control and Mfn1-LKO mice. All values are expressed as mean +/- SEM of n=11 for control mice and n=9 for Mfn1 LKO mice.**

| | Control mice | Mfn1-LKO | *P* value |
|---|---|---|---|
| Mfn1 expression | 1 ± 0.07 | 0.05 ± 0.004 | 1.1*10⁻⁹ |
| (relative to basal) | | | |
| Mfn2 expression | 1 ± 0.09 | 1.37 ± 0.13 | 0.01 |
| (relative to basal) | | | |
| Body weight (g) | 27.90 ± 0.53 | 28.07 ± 0.37 | 0.52 |
| Lean mass (g) | 24.72 ± 0.43 | 24.74 ± 0.26 | 0.67 |
| Fat mass (g) | 2.42 ± 0.24 | 2.76 ± 0.32 | 0.41 |
| Food intake (g/mouse/day) | 2.62 ± 0.23 | 2.41 ± 0.17 | 0.49 |
| Total activity dark phase (arbitrary units) | 1394 ± 107 | 1458 ± 169 | 0.86 |
| Total activity light phase (arbitrary units) | 379 ± 41 | 327 ± 37 | 0.36 |
| Oxygen consumption dark phase (ml/(kg lean mass * hr) | 3611 ± 101 | 3633 ± 119 | 0.89 |
| Oxygen consumption light phase (ml/kg lean mass * hr) | 2913 ± 82 | 2922 ± 122 | 0.95 |
| RER dark phase | 0.98 ± 0.01 | 0.94 ± 0.02 | 0.02 |
| RER light phase | 0.84 ± 0.02 | 0.81 ± 0.01 | 0.03 |
| Glucose oxidation dark phase (mg / (kg lean mass * hr) | 5055 ± 156 | 5507 ± 325 | 0.21 |
| Glucose oxidation light phase (mg / (kg lean mass * hr) | 5346 ± 213 | 5657 ± 274 | 0.39 |
| Lipid oxidation dark phase (mg / (kg lean mass * hr) | 93 ± 31 | 332 ± 99 | 0.02 |
| Lipid oxidation light phase (mg / (kg lean mass * hr) | 753 ± 59 | 897 ± 62 | 0.04 |

**Table 2: Respiration values (pmol/ (s * mg of tissue)) in livers from control and Mfn1-LKO mice. The values are the net respiration rates after substraction of residual respiration remaining after Antimycin-A (complex III inhibitor) addition. All values are expressed as mean +/- SEM of n=11 for control mice and n=9 for Mfn1 LKO mice.**

| | Control mice | Mfn1-LKO | *P* value |
|---|---|---|---|
| Leak (Malate, Pyruvate, Glutamate) | 5.18 ± 0.56 | 7.62 ± 1.17 | 0.08 |
| Complex I (Malate, Pyruvate, Glutamate + ADP) | 50.15 ± 4.13 | 77.82 ± 6.93 | 0.003 |
| Complex I + II (Complex I + Succinate) | 98.50 ± 5.55 | 140.05 ± 9.76 | 0.001 |
| Electron Transport System (ETS) (FCCP) | 161.86 ± 6.18 | 225.56 ± 13.68 | 0.0005 |
| ETS Complex II (ETS + Rotenone) | 102.91 ± 2.66 | 148.23 ± 9.11 | 0.0001 |

### REFERENCES

Canto C, Suarez E, Lizcano JM, Grino E, Shepherd PR, Fryer LG, Carling D, Bertran J, Palacin M, Zorzano A, Guma A (2004) Neuregulin signaling on glucose transport in muscle cells. J Biol Chem 279: 12260-12268
Frezza C, Cipolat S, Scorrano L (2007) Organelle isolation: functional mitochondria from mouse liver, muscle and cultured fibroblasts. Nature protocols 2: 287-295
Holmstrom MH, Iglesias-Gutierrez E, Zierath JR, Garcia-Roves PM (2012) Tissue-specific control of mitochondrial respiration in obesity-related insulin resistance and diabetes. Am J Physiol Endocrinol Metab 302: E731-739
Lagouge M, Argmann C, Gerhart-Hines Z, Meziane H, Lerin C, Daussin F, Messadeq N, Milne J, Lambert P, Elliott P, Geny B, Laakso M, Puigserver P, Auwerx J (2006) Resveratrol improves mitochondrial function and protects against metabolic disease by activating SIRT1 and PGC-1 alpha. Cell 127: 1109-1122
Anila K. Madiraju,Derek M. Erion,Yasmeen Rahimi,Xian-Man Zhang,Demetrios T. Braddock, Ronald A. Albright,Brett J. Prigaro,John L. Wood,Sanjay Bhanot,Michael J. MacDonald, Michael J. Jurczak, Joao-Paulo Camporez, Hui-Young Lee, Gary W. Cline, Varman T. Samuel, Richard G. Kibbey & Gerald I. Shulman (2014) Metformin suppresses gluconeogenesis by inhibiting mitochondrial glycerophosphate dehydrogenase, Nature 510,542-546(26 June 2014)

Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology, or related fields are intended to be within the scope of the following claims.

## Claims

1. A method for identifying an agent that modulates the activity of Mfn1 in a preparation containing an Mfn1 polypeptide or polynucleotide and a candidate agent, comprising detecting whether said candidate agent affects activity of the Mfn1 polypeptide or polynucleotide, wherein the activity of Mfn1 is measured by determining the GTPase activity of Mfn1, and wherein the method is for identifying an agent capable of reducing the activity of Mfn1 but which does not modulate the activity of mitofusin-2 (Mfn2).

2. The method according to claim 1 wherein the method comprises contacting the candidate agent with the Mfn1 polypeptide in the presence of GTP and measuring the hydrolysis of GTP to GDP.

3. The method according to claim 1 wherein the activity of Mfn2 is measured by determining the GTPase activity of Mfn2.

4. An in-vitro method for identifying an agent that reduces the expression or processing of Mfn1 polypeptide, but which does not modulate the expression or processing of Mfn2 polypeptide, comprising contacting cells expressing Mfn1 polypeptide with a candidate agent and monitoring a decrease in expression or processing of said Mfn1 polypeptide.

5. The method according to claim 4 wherein the method is for identifying an agent that reduces the expression or processing of Mfn1 polypeptide comprising contacting cells expressing Mfn1 polypeptide with a candidate agent and monitoring a decrease in expression or processing of said Mfn1 polypeptide.

6. The method according to any preceding claim wherein said method is for identifying an agent that prevents, modulates, reduces or ameliorates symptoms of an Mfn1 associated disease.

7. The method according to any preceding claim wherein said method is for identifying an agent that prevents, reduces or ameliorates the symptoms of a metabolic disease.

8. A method of identifying an agent that prevents, modulates, reduces or ameliorates symptoms of a metabolic disease comprising observing the progression of the metabolic disease in an animal model to which said agent has been administered, wherein the candidate agent is identified using the method of any one of claims 1 to 7.

9. The method according to claim 8 wherein the metabolic disease is selected from the group consisting of insulin resistance, impaired glucose tolerance, impaired fasting glucose and diabetes.

10. The method of any of claims 1 to 9 wherein the agent is an siRNA, shRNA, miRNA, antisense RNA or small molecule.

## Patentansprüche

1. Verfahren zum Identifizieren eines Mittels, das die Aktivität von Mfn1 in einer Zubereitung moduliert, die ein Mfn1-Polypeptid oder -Polynukleotid und ein Kandidatenmittel enthält, umfassend, Feststellen, ob das Kandidatenmittel die Aktivität des Mfn1-Polypeptids oder -Polynukleotids beeinflusst, wobei die Aktivität von Mfn1 durch Bestimmen der GTPase-Aktivität von Mfn1 gemessen wird, und wobei das Verfahren zum Identifizieren eines Mittels dient, das fähig ist, die Aktivität von Mfn1 zu reduzieren, aber nicht die Aktivität von Mitofusin-2 (Mfn2) moduliert.

2. Verfahren nach Anspruch 1, wobei das Verfahren Inkontaktbringen des Kandidatenmittels mit dem Mfn1-Polypeptid unter Vorhandensein von GTP und Messen der Hydrolyse von GTP zu GDP umfasst.

3. Verfahren nach Anspruch 1, wobei die Aktivität von Mfn2 durch Bestimmen der GTPase-Aktivität von Mfn2 gemessen wird.

4. In-vitro-Verfahren zum Identifizieren eines Mittels, das die Expression oder Verarbeitung von Mfn1-Polypeptid reduziert, aber die Expression oder Verarbeitung von Mfn2-Polypeptid nicht moduliert, umfassend Inkontaktbringen von Zellen, die Mfn1-Polypeptid exprimieren, mit einem Kandidatenmittel und Überwachen einer Abnahme der Expression oder Verarbeitung des Mfn1-Polypeptids.

5. Verfahren nach Anspruch 4, wobei das Verfahren zum Identifizieren eines Mittels dient, das die Expression oder Verarbeitung von Mfn1-Polypeptid reduziert, umfassend Inkontaktbringen von Zellen, die Mfn1-Polypeptid exprimieren, mit einem Kandidatenmittel und Überwachen einer Abnahme der Expression oder Verarbeitung des Mfn1-Polypeptids.

6. Verfahren nach einem der vorstehenden Ansprüche, wobei das Verfahren zum Identifizieren eines Mittels dient, das Symptome einer Mfn1-assoziierten Erkrankung verhindert, moduliert, reduziert oder mildert.

7. Verwendung nach einem der vorstehenden Ansprüche, wobei das Verfahren zum Identifizieren eines Mittels dient, das die Symptome einer Stoffwechselerkrankung verhindert, reduziert oder mildert.

8. Verfahren zum Identifizieren eines Mittels, das Symptome einer Stoffwechselerkrankung verhindert, moduliert, reduziert oder mildert, umfassend Beobachten des Fortschreitens der Stoffwechselerkrankung in einem Tiermodell, dem das Mittel verabreicht wurde, wobei das Kandidatenmittel unter Verwendung des Verfahrens nach einem der Ansprüche 1 bis 7 identifiziert wird.

9. Verfahren nach Anspruch 8, wobei die Stoffwechselerkrankung ausgewählt wird aus der Gruppe bestehend aus Insulinresistenz, beeinträchtigter Glucosetoleranz, beeinträchtigtem Nüchternblutzucker und Diabetes.

10. Verfahren nach einem der Ansprüche 1 bis 9, wobei das Mittel eine siRNA, shRNA, miRNA, Antisense-RNA oder ein kleines Molekül ist.

## Revendications

1. Procédé pour identifier un agent qui module l'activité de Mfn1 dans une préparation contenant un polypeptide ou polynucléotide de Mfn1 et un agent candidat, comprenant la détection si ledit agent candidat affecte l'activité du polypeptide ou polynucléotide de Mfn1, dans lequel l'activité de Mfn1 est mesurée en déterminant l'activité GTPase du Mfn1, et dans lequel le procédé est destiné à identifier un agent apte à réduire l'activité de Mfn1 mais qui ne module pas l'activité de la mitofusine 2 (Mfn2).

2. Procédé selon la revendication 1 dans lequel le procédé comprend la mise en contact de l'agent candidat avec le polypeptide de Mfn1 en présence de GTP et la mesure de l'hydrolyse de GTP en GDP.

3. Procédé selon la revendication 1 dans lequel l'activité de Mfn2 est mesurée en déterminant l'activité GTPase de Mfn2.

4. Procédé in vitro pour identifier un agent qui réduit l'expression ou le traitement du polypeptide de Mfn1, mais qui ne module l'expression ou le traitement du polypeptide de Mfn2, comprenant la mise en contact de cellules exprimant le polypeptide de Mfn1 avec un agent candidat et la surveillance d'une diminution de l'expression ou du traitement dudit polypeptide de Mfn1.

5. Procédé selon la revendication 4 dans lequel le procédé est destiné à identifier un agent qui réduit l'expression ou le traitement d'un polypeptide de Mfn1 comprenant la mise en contact des cellules exprimant le polypeptide de Mfn1 avec un agent candidat et la surveillance d'une diminution de l'expression ou du traitement dudit polypeptide de Mfn1.

6. Procédé selon l'une quelconque des revendications précédentes dans lequel ledit procédé est destiné à identifier un agent qui prévient, module, réduit ou améliore les symptômes d'une maladie associée à Mfn1.

7. Procédé selon l'une quelconque des revendications précédentes dans lequel ledit procédé est destiné à identifier un agent qui prévient, réduit ou améliore les symptômes d'une maladie métabolique.

8. Procédé d'identification d'un agent qui prévient, module, réduit ou améliore les symptômes d'une maladie métabolique comprenant l'observation de la progression de la maladie métabolique dans un modèle animal auquel ledit agent a été administré, dans lequel l'agent candidat est identifié en utilisant le procédé selon l'une quelconque des revendications 1 à 7.

9. Procédé selon la revendication 8 dans lequel la maladie métabolique est choisie dans le groupe constitué de résistance à l'insuline, intolérance au glucose, hyperglycémie modérée à jeun et diabète.

10. Procédé selon l'une quelconque des revendications 1 à 9 dans lequel l'agent est un petit ARN interférent, un ARNsh, un micro-ARN, un ARN antisens ou une petite molécule.
